# EUROPÄISCHE PATENTSCHRIFT

(11) **EP 4 015 079 B1**
(45) Veröffentlichungstag und Bekanntmachung des Hinweises auf die Patenterteilung: **05.02.2025**
(21) Anmeldenummer: 20215551.1
(22) Anmeldetag: 18.12.2020
(51) Int. Cl.: B01J 23/96, B01J 23/46, B01J 38/14, B01J 38/52, C07C 67/303

(54) **VERFAHREN ZUR REGENERIERUNG VON HYDRIERKATALYSATOREN**
METHOD FOR REGENERATING HYDROGENATION CATALYSTS
PROCÉDÉ DE RÉGÉNÉRATION DES CATALYSEURS D'HYDROGÈNE

(43) Veröffentlichungstag der Anmeldung: 22.06.2022
(73) Patentinhaber: Evonik Oxeno GmbH & Co. KG, 45772 Marl (DE)
(72) Erfinder: KRAFT, Johannes, 45770 Marl (DE); ALTMANN, Lena, 46282 Dorsten (DE); ANTON, Johan, 46284 Dorsten (DE); GRASS, Michael, 45721 Haltern am See (DE)
(74) Vertreter: Evonik Patent Association

(56) Entgegenhaltungen:
- WO-A1-02/100536
- WO-A2-2006/136541
- BE-A- 882 279

## Beschreibung

Die vorliegende Erfindung betrifft ein Verfahren zur Regenerierung eines für die Kernhydrierung eines aromatischen Esters eingesetzten Katalysators, wobei für die Regenerierung ein Gasstrom eingesetzt wird, der eine bestimme Menge an Sauerstoff enthält.

Die Hydrierung von Aromaten und insbesondere von aromatischen Estern, bei der der aromatische Ring hydriert wird, ist bekannt und wird auch als Kernhydrierung bezeichnet. Bei einer solchen Kernhydrierung werden beispielsweise Übergangsmetall-enthaltende Katalysatoren eingesetzt. Geeignete Katalysatoren sind dem Fachmann bekannt. Entsprechende Hydrierverfahren werden auch großindustriell eingesetzt.

Mit fortschreitender Dauer der Kernhydrierung können die eingesetzten Katalysatoren an Aktivität verlieren, sei es durch Blockieren, Verlust oder Vergiften der aktiven Zentren des Katalysators. Ist der Aktivitätsverlust zu hoch und/oder lässt sich das Hydrierverfahren nicht mehr ausreichend wirtschaftlich betreiben, muss die Aktivität des Katalysators gesteigert werden. Dies geschieht mittels eines Regenerierverfahrens, bei dem der Katalysator von Ablagerungen und Anhaftungen befreit wird. Möglich ist dabei eine Kalzination des Katalysators, bei dem die Ablagerungen bei hohen Temperaturen (> 200 °C) entfernt werden, was jedoch für bestimmte Katalysatoren oder auch rein energetisch aufgrund der hohen Temperaturen problematisch sein kann.

Katalysatoren können aber auch durch Überleiten eines Gasstroms regeneriert werden, durch den die Ablagerungen mitgerissen und dadurch entfernt werden. Ein solches Verfahren zur Regenerierung von Rutheniumkatalysatoren wird beispielsweise in der WO 2008/015103 A1 beschrieben. Die dort offenbarte Regenerierung zeichnet sich dadurch aus, dass der Katalysator mit einem Inertgas gespült wird, bis der Katalysator seine Aktivität teilweise oder sogar vollständig wiedererlangt hat. Als Grund für die dort anhand der Benzolhydrierung beschriebene regenerierende Wirkung wird die Entfernung von Wasser, d. h. das Trocknen des Katalysators genannt.

Die WO 02/100536 A1 ein Verfahren zur Hydrierung mindestens einer aromatischen Verbindung durch Inkontaktbringen mit einem Wasserstoff enthaltenden Gas in Gegenwart eines Katalysators, der als Aktivmetall Ruthenium alleine oder zusammen mit mindestens einem weiteren Metall der I., VII. oder VIII. Nebengruppe des Periodensystems, aufgebracht auf ein Trägermaterial auf Basis von amorphem Siliziumdioxid, umfasst.

Die WO 2006/136541 A1 offenbart einen Schalenkatalysator, der Ruthenium alleine oder zusammen mit mindestens einem weiteren Metall der Nebengruppen IB, VIIB oder VIII des Periodensystems der Elemente (CAS-Version) enthält und einem Träger enthaltend Siliziumdioxid aufgebracht wird und ein Verfahren zur Hydrierung einer carbocyclischen aromatischen Gruppe zu der entsprechenden carbocyclischen aliphatischen Gruppen unter Verwendung des Schalenkatalysators.

Die BE 882.279 A offenbart weiterhin ein Verfahren zur Reaktivierung eines Katalysators mit einem Gas, das einen Volumenanteil von Sauerstoff im Bereich von 1 bis 100% aufweist, zur Hydrierung von Zuckern, der auf mindestens einem der sechs Metalle der Platingruppe, nämlich Ruthenium, Rhodium, Palladium, Osmium, Iridium und Platin, basiert.

Das bekannte Verfahren weist jedoch das Problem auf, dass der Regeneriereffekt zu gering sein kann, insbesondere dann, wenn nicht nur Wasser vom Katalysator entfernt werden muss oder sich nicht nur Wasser, sondern auch andere, die Hydrieraktivität senkende, Substanzen auf dem Katalysator befinden.

Demzufolge bestand die Aufgabe der vorliegenden Erfindung darin, Verfahren zur Regenerierung eines für die Kernhydrierung eines Aromaten, insbesondere eines aromatischen Esters, eingesetzten Katalysators bereitzustellen, mit dem eine akzeptable Aktivität schneller und besser erreicht werden kann.

Die Aufgabe wird durch das in Anspruch 1 genannte Verfahren gelöst. Bevorzugte Ausführungsformen sind in den Unteransprüchen angegeben. Das erfindungsgemäße Verfahren ist ein Verfahren zur Regenerierung eines für die Kernhydrierung eines Esters einer Benzolcarbonsäure, einer Benzoldicarbonsäure, einer Benzoltricarbonsäure oder einer Benzoltetracarbonsäure eingesetzten Katalysators, der mindestens ein Übergangsmetall aus der Gruppe, bestehend aus Eisen, Ruthenium, Nickel, Rhodium, Platin, Palladium oder Mischungen davon, auf einem Trägermaterial umfasst, in mindestens einem Reaktor, wobei die Regenerierung
- durch Überleiten eines Gasstroms, der einen Gehalt an Sauerstoff von 380 ppm bis 9000 ppm aufweist, über den zu regenerierenden Katalysator;
- bei einer Temperatur von 15 bis 170 °C, vorzugsweise von 25 bis 150 °C, besonders bevorzugt von 25 bis 120 °C, ganz besonders bevorzugt 25 bis 110 °C; und
- ohne Ausbau des Katalysators aus dem mindestens einen Reaktor
durchgeführt wird.

Durch das erfindungsgemäße Verfahren, bei dem geringe Mengen Sauerstoff im Gasstrom, dem Regeneriermedium, vorhanden sind, kann eine vorteilhafte Regenerierung des Hydrierkatalysators erreicht werden. Durch die Regenerierung wird die Katalysatoraktivität verbessert, was gemäß der vorliegenden Erfindung bereits bei den geringen Sauerstoffkonzentrationen erfolgen kann. Die hier beschriebene Regenerierung ermöglicht, dass die Kernhydrierung, wodurch die eigentlichen Wertprodukte hergestellt werden, anschließend bei gesteigerten Umsätzen wieder in Betrieb genommen werden und somit effektiver durchgeführt werden kann. Gleichzeitig verhindert die eher geringe Sauerstoffkonzentration, dass es durch Oxidationsprozesse zu stark (gegebenenfalls auch nur lokal) erhöhten Temperaturen im Reaktor kommt, die den Katalysator schädigen oder im schlimmsten Fall zu Schäden am Reaktor führen können.

Die hier beschriebene Kernhydrierung von aromatischen Estern wird üblicherweise in mindestens einem Reaktor durchgeführt, d. h. die Kernhydrierung kann in einem oder mehreren Reaktoren erfolgen, die jeweils einen geeigneten Katalysator enthalten. Die erfindungsgemäße Regenerierung des eingesetzten Katalysators kann in jedem der vorliegenden Reaktoren erfolgen. Die Regenerierung kann bei Vorliegen von mehreren Reaktoren für jeden Reaktor zeitlich versetzt oder gleichzeitig erfolgen, wobei eine gleichzeitige Regenerierung aller Katalysatoren in allen vorliegenden Reaktoren bevorzugt ist.

Erfindungsgemäß eignet sich das Verfahren für alle bei der Kernhydrierung von aromatischen Estern eingesetzten Katalysatoren. Der Katalysator umfasst mindestens ein Übergangsmetall auf einem Trägermaterial oder besteht aus mindestens einem Übergangsmetall auf einem Trägermaterial. Geeignete Katalysatoren sind dem Fachmann aber auch geläufig und beispielsweise der WO 03/103830 A1 zu entnehmen.

Das Übergangsmetall des zu regenerierenden Katalysators ist ein Metall, welches aus der Gruppe, bestehend aus Eisen, Ruthenium, Nickel, Rhodium, Platin, Palladium oder Mischungen davon, ausgewählt wird. Ruthenium ist das in der vorliegenden Erfindung besonders bevorzugte Übergangsmetall des eingesetzten Katalysators. Der Gehalt an Übergangsmetall im zu regenerierenden Katalysator beträgt im Allgemeinen 0,1 bis 30 Massen-%. Der Rutheniumgehalt, berechnet als Metall, liegt vorzugsweise im Bereich von 0,1 bis 10 Massen-%, insbesondere im Bereich von 0,3 bis 5 Massen-%, ganz besonders im Bereich zwischen 0,4 und 2,5 Massen-%.

Das Trägermaterial, auf dem sich das Übergangsmetall befindet, wird vorzugsweise aus der Gruppe, bestehend aus Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon, ausgewählt. Bevorzugte Trägermaterialien sind Aluminiumoxid, Siliciumdioxid, Titandioxid und Mischung davon. Zusätzlich können diese Trägermaterialien Alkalimetalle, Erdalkalimetalle und/oder Schwefel enthalten. In einer besonders bevorzugten Ausführungsform der vorliegenden Erfindung ist der zu regenerierende Katalysator ein Schalenkatalysator.

Als Regeneriermedium wird erfindungsgemäß ein Gasstrom eingesetzt. Dieser Gasstrom besteht vorzugsweise aus einem Inertgas und Sauerstoff, wobei der Gasstrom einen Gehalt an Sauerstoff von 380 ppm bis 9000 ppm aufweist. Das Inertgas kann Stickstoff, Helium, Neon, Argon, Kohlendioxid und eine Mischung daraus sein. Besonders bevorzugt wird Stickstoff als das Inertgas eingesetzt. Der Gasstrom bei der Regenerierung kann in gleicher oder umgekehrter Strömungsrichtung, bezogen auf die Strömungsrichtung bei der Kernhydrierung, über den Katalysator geleitet werden, vorzugsweise in gleicher Strömungsrichtung.

Bei Vorliegen mehrerer Hydrierreaktoren kann das Regeneriermedium ausschließlich zum ersten Reaktor zugeführt werden. Das Regeneriermedium würde dann vom ersten Reaktor auch zu dem oder zu den nachfolgenden Reaktor(en) geführt werden, um auch die dortigen Katalysatoren zu regenerieren. Es ist aber bevorzugt, dass das Regeneriermedium bei Vorliegen mehrerer Reaktoren jeweils zu jedem einzelnen Reaktor geführt wird. Das Regeneriermedium wird also direkt zu jedem einzelnen Reaktor geführt und durchläuft nicht erst die vorherigen Reaktoren bzw. den vorherigen Reaktor.

Die Regenerierung wird anspruchsgemäß bei einer Temperatur von 15 bis 170 °C, vorzugsweise von 25 bis 150 °C, besonders bevorzugt von 25 bis 120 °C durchgeführt. Wichtig in diesem Zusammenhang ist, dass hier kein Abbrand des Katalysators erfolgt, bei dem die Verunreinigungen bei hohen Temperaturen entfernt werden. Der Druck bei der Regenerierung ist vorzugsweise im Bereich von 0,5 bis 200 bar, vorzugsweise 1 bis 110 bar.

Es hängt von verschiedenen Faktoren ab, wie lange die vorliegend beschriebene Regenerierung durchgeführt werden muss, um die Katalysatoraktivität in ausreichendem Maße zu verbessern. Beispiele für diese Faktoren sind die Art und Menge der Verunreinigungen, die Beschaffenheit der Reaktoren (Größe, Durchmesser) oder die Beschaffenheit der Katalysatorpartikel (insbesondere Größe, Form, Oberfläche, Porenstruktur, Metallbeladung sowie Eindringtiefe des Metalls im Träger). In einer bevorzugten Ausführungsform der vorliegenden Erfindung beträgt die Dauer der Regenerierung mindestens 24 Stunden. Wird die Regenerierung zu früh beendet, kann es vorkommen, dass der Katalysator schon nach relativ kurzem Einsatz in der Kernhydrierung erneut regeneriert werden muss.

Die erfindungsgemäße Regenerierung erfolgt bei einem in der Kernhydrierung eines aromatischen Esters eingesetzten Katalysators. Der aromatische Ester ist ein Ester einer Benzolcarbonsäure, ein Ester einer Benzoldicarbonsäure, ein Ester einer Benzoltricarbonsäure oder ein Ester einer Benzoltetracarbonsäure, vorzugsweise ein Ester der Benzoldicarbonsäure oder der Benzoltricarbonsäure. Dazu zählen Phthalate, Isophthalate, Terephthalate und Trimellitate. Bevorzugt sind Ester der Benzoldicarbonsäure, also Phthalate, Isophthalate und Terephthalate. Davon sind besonders die C8- bis C10-Alkylester, insbesondere die C8- bis C10-Alkylester der Phthalsäure (z. B. Dioctylphthalat, Diethylhexylphthalat, Diisononylphthalat, Dipropylheptylphthalat) oder die C8- bis C10-Alkylester der Terephthalsäure (z. B. Dioctylterephthalat, Diethylhexylterephthalat, Diisononylterephthalat, Dipropylheptylterephthalat) bevorzugt.

Die Kernhydrierung wird bevorzugt in flüssiger Phase durchgeführt. Die Kernhydrierung kann an suspendierten oder stückigen im Festbett angeordneten Katalysatoren kontinuierlich oder diskontinuierlich durchgeführt werden. Im erfindungsgemäßen Verfahren wird eine kontinuierliche Kernhydrierung an einem im Festbett angeordnetem Katalysator, bei dem sich unter Reaktionsbedingungen die Produkt/Eduktphase hauptsächlich im flüssigen Zustand befindet, bevorzugt. Vorzugsweise wird/werden der/die Reaktor(en) als Rieselbettreaktor(en) betrieben, der/die vollständig oder teilweise geflutet sein kann/können.

Wenn die Kernhydrierung kontinuierlich an einem im Festbett angeordneten Katalysator durchgeführt wird, ist es zweckmäßig, den Katalysator vor der erstmaligen Durchführung der Kernhydrierung in die aktive Form zu überführen. Nach einer Regenerierung ist eine solche Aktivierung nicht unbedingt erforderlich. Die Aktivierung kann durch Reduktion des Katalysators mit Wasserstoff-haltigen Gasen nach einem Temperaturprogramm erfolgen. Dabei kann die Reduktion gegebenenfalls in Gegenwart einer flüssigen Phase, die über den Katalysator rieselt, durchgeführt werden. Als flüssige Phase kann dabei ein Lösemittel oder das Hydrierprodukt eingesetzt werden. Bei entsprechenden Hydrierbedingungen ist auch ein Verzicht auf eine derartige Aktivierung möglich, da diese auch unter Reaktionsbedingungen erfolgt.

Für die Kernhydrierung können unterschiedliche Verfahrensvarianten gewählt werden. Es kann adiabatisch, polytrop oder praktisch isotherm, d. h. mit einem Temperaturanstieg von typischerweise kleiner als 10 °C, ein- oder mehrstufig durchgeführt werden. Im letzteren Fall kann man alle Reaktoren, zweckmäßig Rohrreaktoren, adiabatisch oder praktisch isotherm sowie einen oder mehrere adiabatisch und die anderen praktisch isotherm betreiben. Weiterhin ist es möglich, die aromatischen Polycarbonsäureester im geraden Durchgang oder unter Produktrückführung zu hydrieren. Es ist auch möglich, dass zwei Reaktoren vorhanden sind, wobei der erste Reaktor unter Produktrückführung und der zweite Reaktor im geraden Durchgang betrieben wird. Möglich ist auch eine Durchführung der Kernhydrierung als Trickle-Bed. Der Reaktor kann dabei auch teilweise oder vollständig geflutet sein.

Die Kernhydrierung kann in der Flüssig/Gas-Mischphase oder in der Flüssigphase in Dreiphasenreaktoren im Gleichstrom durchgeführt werden, wobei das Hydriergas in an sich bekannter Weise im flüssigen Edukt/Produktstrom verteilt wird. Im Interesse einer gleichmäßigen Flüssigkeitsverteilung, einer verbesserten Reaktionswärmeabfuhr und einer hohen Raum-ZeitAusbeute werden die Reaktoren vorzugsweise mit hohen Flüssigkeitsbelastungen von 15 bis 120, insbesondere von 25 bis 80 m³ pro m² Querschnitt des leeren Reaktors und Stunde betrieben. Wird ein Reaktor im geraden Durchgang betrieben, so kann die spezifische Katalysatorbelastung (LHSV) Werte zwischen 0,1 und 10 h⁻¹ annehmen.

Die Kernhydrierung kann in Ab- oder vorzugsweise in Anwesenheit eines Lösungsmittels durchgeführt werden. Als Lösemittel können alle Flüssigkeiten eingesetzt werden, die mit dem Edukt und Produkt eine homogene Lösung bilden, sich unter Hydrierbedingungen inert verhalten und sich leicht vom Produkt abtrennen lassen. Das Lösemittel kann auch ein Gemisch mehrerer Stoffe sein und gegebenenfalls Wasser enthalten.

Beispielsweise können folgende Stoffe als Lösemittel eingesetzt werden: Geradkettige oder cyclische Ether, wie beispielsweise Tetrahydrofuran oder Dioxan sowie aliphatische Alkohole, in denen der Alkylrest 1 bis 13 Kohlenstoffatome aufweist. Bevorzugt verwendbare Alkohole sind beispielsweise Isopropanol, n-Butanol, Isobutanol, n-Pentanol, 2-Ethylhexanol, Nonanole, technische Nonanolgemische, Decanol, technische Decanolgemische, Tridecanole.

Bei Einsatz von Alkoholen als Lösemittel kann es zweckmäßig sein, denjenigen Alkohol oder dasjenige Alkoholgemisch zu verwenden, das bei der Verseifung des Produkts entstehen würde. Dadurch ist die Nebenproduktbildung durch Umesterung ausgeschlossen. Ein weiteres bevorzugtes Lösemittel ist das Hydrierprodukt selbst.

Durch die Verwendung eines Lösemittels kann die Aromatenkonzentration im Reaktorzulauf begrenzt werden, wodurch eine bessere Temperaturkontrolle im Reaktor erreicht werden kann. Dies kann eine Minimierung von Nebenreaktionen und somit eine Erhöhung der Produktausbeute zu Folge haben. Bevorzugt liegt der Aromatengehalt im Reaktorzulauf zwischen 1 und 35 %, insbesondere zwischen 5 und 25 %. Der gewünschte Konzentrationsbereich kann bei Reaktoren, die in Schlaufenfahrweise betrieben werden, durch die Zirkulationsrate (Mengenverhältnis von rückgeführtem Hydrieraustrag zu Edukt) eingestellt werden.

Die Kernhydrierung kann in einem Druckbereich von 20 bis 300 bar, vorzugsweise 40 bis 200 bar durchgeführt werden. Die Hydriertemperaturen liegen vorzugsweise im Bereich von 60 bis 200°C, insbesondere im Bereich von 80 bis 180 °C.

Als Hydriergase können beliebige Wasserstoff-haltige Gasgemische, die keine schädlichen Mengen an Katalysatorgiften wie beispielsweise Kohlenmonoxid oder Schwefelwasserstoff enthalten, eingesetzt werden. Die Inertgasbestandteile können beispielsweise Stickstoff oder Methan sein. Bevorzugt wird Wasserstoff in einer Reinheit von größer 95 %, insbesondere größer 98 % eingesetzt.

Die Erfindung wird nachfolgend anhand von Beispielen erläutert. Diese Beispiele zeigen exemplarische Ausführungsformen und sind nicht einschränkend zu verstehen.

### Beispiel 1 - gleiche Sauerstoffkonzentration

Für die vorliegenden Versuche musste zunächst reproduzierbar ein Katalysator erzeugt werden, der anschließend zu regenerieren war. Im vorliegenden Fall wurde dazu kontinuierlich Diisononylterephthalat (DINT) hydriert. Die Kernhydrierung von DINT erfolgte in einem Rohrreaktor im Kreislaufbetrieb. Der Kreislaufrohrreaktor wurde im Gleichstrom mit Flüssigphase (DINT und Hydrierprodukt) und Gasphase (Wasserstoff) im Rieselbett durchströmt. Als Hydrierkatalysator wurde in Beispiel 1 ein handelsüblicher Rutheniumkatalysator (Specialyst^{®} 102: 1% Ru auf einem TiO₂-Träger, Fa. Evonik Operations GmbH) verwendet. Dieser wurde in äquivalenter Menge mit Inertmaterial (TiO₂) verdünnt und in dem Rohrreaktor mit einem Innendurchmesser von 40 mm und einer Länge von 378 mm eingesetzt. Die in der Kernhydrierung eingesetzte Feedrate an DINT betrug stets 130 g/h, der Kreislaufstrom 80 L/h. Die H₂-Regelung erfolgt über eine konstante Abgasfahrweise mit einem Abgasstrom von 0,5 L/h. Die Versuche wurden jeweils bei einem Anlagendruck von 100 bar sowie einer Rohrreaktortemperatur von 110 °C durchgeführt. Die DINT Konzentration wurde mittels inline Ramananalytik erfasst. Mit der Zeit verringerte sich kontinuierlich der DINT-Umsatz. Nach etwa 3700 bis 5700 Stunden wurde dann jeweils eine Regenerierung vorgenommen.

Vor der Regenerierung wurden die Kreislaufpumpe und die Zufuhr der Reaktanden gestoppt und die Flüssigkeiten aus dem Kreislaufreaktor abgelassen. Anschließend wurden die jeweiligen Regenerierungsbedingungen (z. B. Druck, Temperatur) eingestellt und die Regenerierung für einen bestimmten Zeitraum durchgeführt (s. Tabelle 1). Nach der Regenerierung wurden Kreislaufpumpe und die Zufuhr der Reaktanden wieder gestartet, um wieder eine Kernhydrierung, wie im vorherigen Absatz beschrieben, durchzuführen. Als Grundlage für die Bewertung der Effektivität der Regenerierung wurden die Probennahme unmittelbar vor Ausschalten der Reaktandenzufuhr (DINT Umsatz vor Regenerierung) und ca. 20 bis 40 h nach Wiederanlauf der Reaktandenzufuhr (DINT Umsatz nach Regenerierung) verwendet. Die Ergebnisse sind in der nachfolgenden Tabelle 1 aufgeführt.

**Tabelle1: Versuchsdaten zu Beispiel 1**

| **Regenerierung** | **Druck / bar** | **T/ °C** | **Dauer / Tage** | **O₂**-**Konzentration / ppm** | **Umsatz Kreislaufreaktor / %** | | |
|---|---|---|---|---|---|---|---|
| | | | | | **Vorher** | **Nachher** | **Differenz** |
| 1 | 100 | 110 | 3 | 400 | 91,0 | 94,3 | 3,3 |
| 2 | 10 | 110 | 3 | 400 | 90,6 | 94,1 | 3,5 |
| 3 | 10 | 110 | 1 | 400 | 90,8 | 93,9 | 3,1 |

Aus der Tabelle 1 ist zu sehen, dass weder der Druck noch die Dauer der Regenerierung einen signifikanten Einfluss auf die Regenerierung haben.

### Beispiel 2 - unterschiedliche Sauerstoffkonzentrationen

Die Versuche für Beispiel 2 wurden in ähnlicher Weise wie die Versuche für Beispiel 1 durchgeführt. Im Folgenden sind daher nur die Unterschiede zu dem Verfahren in Beispiel 1 aufgeführt. Als Hydrierkatalysator wurde ein Rutheniumkatalysator mit größerer Menge an Ru eingesetzt (2% Ru auf TiO₂-Träger). Die in der Kernhydrierung eingesetzte Feedrate an DINT betrug immer 180 g/h und es wurde bei einer Temperatur von 92,5 °C hydriert. Im Unterschied zu Beispiel 1 wurden hier neben Temperatur und Druck auch die Sauerstoffkonzentrationen bei der Regeneration variiert. Zur Vermeidung von Problemen (Knallgas) wurde der Reaktor vor den Regenerierungsversuchen mit höherer Oz-Konzentration zunächst mit N₂ inertisiert. Die Ergebnisse sind in Tabelle 2 gezeigt.

**Tabelle 2: Versuchsdaten zu Beispiel 2**

| **Regenerierung** | **Druck / bar** | **T/ °C** | **Dauer/ Tage** | **O₂**-**Konzentration / ppm** | **Umsatz Kreislaufreaktor / %** | | |
|---|---|---|---|---|---|---|---|
| | | | | | **Vorher** | **Nachher** | **Differenz** |
| 1 | 100 | 110 | 3 | 400 | 90 | 92,5 | 2,5 |
| 2* | 10 | 110 | 4 | 36 | 89,4 | 89,8 | 0,4 |
| 3* | 1 | RT | 1 | 20000 | 88,2 | 90,2 | 2 |
| 4* | 100 | RT | 1 | 20000 | 88,5 | 90,6 | 2,1 |
| 5 | 1 | 110 | 1 | 5000 | 88,1 | 91,0 | 2,9 |
| 6 | 100 | RT | 1 | 5000 | 86,8 | 88,7 | 1,9 |

| | | | | | | | |
|---|---|---|---|---|---|---|---|
| * nicht erfindungsgemäß | | | | | | | |

Aus Tabelle 2 wird ersichtlich, dass bei sehr geringen Sauerstoffkonzentrationen schlechtere Regenerierungen erreicht werden konnten. Bei hohen Sauerstoffkonzentrationen ist die Regenerierung auch in kurzer Zeit besser, hier ist jedoch zu beachten, dass bei so hohen Konzentrationen das Risiko einer Kalzination bzw. eines Abbrands des Katalysators besteht, weswegen entweder nur geringe Temperaturen oder ein geringerer Druck eingesetzt werden konnten. Noch höhere Sauerstoffkonzentrationen (> 20000 ppm) haben sich im vorliegenden Fall aus Sicherheitsgründen als nicht zielführend erwiesen.

## Patentansprüche

1. Verfahren zur Regenerierung eines für die Kernhydrierung eines Esters einer Benzolcarbonsäure, einer Benzoldicarbonsäure, einer Benzoltricarbonsäure oder einer Benzoltetracarbonsäure eingesetzten Katalysators, der mindestens ein Übergangsmetall aus der Gruppe, bestehend aus Eisen, Ruthenium, Nickel, Rhodium, Platin, Palladium oder Mischungen davon, auf einem Trägermaterial umfasst, in mindestens einem Reaktor, wobei die Regenerierung
- durch Überleiten eines Gasstroms, der einen Gehalt an Sauerstoff von 380 ppm bis 9000 ppm aufweist, über den zu regenerierenden Katalysator;
- bei einer Temperatur von 15 bis 170 °C; und
- ohne Ausbau des Katalysators aus dem mindestens einen Reaktor
durchgeführt wird.

2. Verfahren nach einem der vorhergehenden Ansprüche, wobei die Regenerierung bei einer Temperatur von 25 bis 150 °C durchgeführt wird.

3. Verfahren nach Anspruch 1 oder 2, wobei das Trägermaterial aus der Gruppe, bestehend aus Aktivkohle, Siliciumcarbid, Aluminiumoxid, Siliciumdioxid, Alumosilikat, Titandioxid, Zirkoniumdioxid, Magnesiumoxid, Zinkoxid oder Mischungen davon, ausgewählt wird.

4. Verfahren nach einem der Ansprüche 1 bis 3, wobei der eingesetzte Katalysator ein Schalenkatalysator ist.

5. Verfahren nach einem der vorhergehenden Ansprüche, wobei der Gasstrom ein Inertgas, vorzugsweise Stickstoff, Helium, Neon, Argon, Neon, Kohlendioxid und Mischungen daraus, ist, der einen Gehalt an Sauerstoff nach Anspruch 1 aufweist.

6. Verfahren nach Anspruch 1, wobei der aromatische Ester ein Ester der Benzoldicarbonsäure oder der Benzoltricarbonsäure, bevorzugt ein Ester der Benzoldicarbonsäure ist.

7. Verfahren nach Anspruch 6, wobei der aromatische Ester ein C8- bis C10-Alkylester der Phthalsäure oder ein C8- bis C10-Alkylester der Terephthalsäure ist.

8. Verfahren nach einem der vorhergehenden Ansprüche, wobei die Regenerierung bei einem Druck im Bereich von 0,5 bis 200 bar durchgeführt wird.

9. Verfahren nach einem der vorhergehenden Ansprüche, wobei die Dauer der Regenerierung mindestens 24 Stunden beträgt.

10. Verfahren nach einem der vorhergehenden Ansprüche, wobei der Gasstrom bei der Regenerierung in gleicher oder umgekehrter Strömungsrichtung, bezogen auf die Strömungsrichtung bei der Hydrierung, über den Katalysator geleitet wird, vorzugsweise in gleicher Strömungsrichtung.

## Claims

1. Process for regenerating a catalyst which is used for the ring hydrogenation of an ester of a benzenecarboxylic acid, of a benzenedicarboxylic acid, of a benzenetricarboxylic acid or of a benzenetetracarboxylic acid and comprises at least one transition metal from the group consisting of iron, ruthenium, nickel, rhodium, platinum, palladium and mixtures thereof on a support material in at least one reactor, wherein the regeneration is conducted
- by passing a gas stream having an oxygen content of 380 ppm to 9000 ppm over the catalyst to be regenerated;
- at a temperature of 15 to 170°C; and
- without deinstalling the catalyst from the at least one reactor.

2. Process according to any of the preceding claims, wherein the regeneration is conducted at a temperature of 25°C to 150°C.

3. Process according to Claim 1 or 2, wherein the support material is selected from the group consisting of activated carbon, silicon carbide, aluminium oxide, silicon dioxide, aluminosilicate, titanium dioxide, zirconium dioxide, magnesium oxide, zinc oxide and mixtures thereof.

4. Process according to any of Claims 1 to 3, wherein the catalyst used is an eggshell catalyst.

5. Process according to any of the preceding claims, wherein the gas stream is an inert gas, preferably nitrogen, helium, neon, argon, carbon dioxide and mixtures thereof, having an oxygen content according to Claim 1.

6. Process according to Claim 1, wherein the aromatic ester is an ester of benzenedicarboxylic acid or of benzenetricarboxylic acid, preferably an ester of benzenedicarboxylic acid.

7. Process according to Claim 6, wherein the aromatic ester is a C8- to C10-alkyl ester of phthalic acid or a C8- to C10-alkyl ester of terephthalic acid.

8. Process according to any of the preceding claims, wherein the regeneration is conducted at a pressure in the range of 0.5 to 200 bar.

9. Process according to any of the preceding claims, wherein the duration of the regeneration is at least 24 hours.

10. Process according to any of the preceding claims, wherein the gas stream in the regeneration is guided over the catalyst in the same or in the reverse flow direction, based on the flow direction in the hydrogenation, preferably in the same flow direction.

## Revendications

1. Procédé de régénération d'un catalyseur utilisé pour l'hydrogénation du noyau d'un ester d'un acide benzènecarboxylique, d'un acide benzènedicarboxylique, d'un acide benzènetricarboxylique ou d'un acide benzènetétracarboxylique, qui comprend au moins un métal de transition du groupe constitué par le fer, le ruthénium, le nickel, le rhodium, le platine, le palladium ou leurs mélanges, sur un matériau support, dans au moins un réacteur, la régénération étant effectuée
- par passage d'un flux gazeux, qui présente une teneur en oxygène de 380 ppm à 9000 ppm sur le catalyseur à régénérer ;
- à une température de 15 à 170°C ; et
- sans démontage du catalyseur dudit au moins un réacteur.

2. Procédé selon l'une des revendications précédentes, la régénération étant effectuée à une température de 25 à 150°C.

3. Procédé selon la revendication 1 ou 2, le matériau support étant choisi dans le groupe constitué par le charbon actif, le carbure de silicium, l'oxyde d'aluminium, le dioxyde de silicium, l'aluminosilicate, le dioxyde de titane, le dioxyde de zirconium, l'oxyde de magnésium, l'oxyde de zinc ou leurs mélanges.

4. Procédé selon l'une des revendications 1 à 3, le catalyseur utilisé étant un catalyseur enrobé.

5. Procédé selon l'une des revendications précédentes, le flux gazeux étant un gaz inerte, de préférence l'azote, l'hélium, le néon, l'argon, le néon, le dioxyde de carbone ou leurs mélanges, qui présente une teneur en oxygène selon la revendication 1.

6. Procédé selon la revendication 1, l'ester aromatique étant un ester de l'acide benzènedicarboxylique ou de l'acide benzènetricarboxylique, de préférence un ester de l'acide benzènedicarboxylique.

7. Procédé selon la revendication 6, l'ester aromatique étant un ester C8-C10-alkylique de l'acide phtalique ou un ester C8-C10-alkylique de l'acide téréphtalique.

8. Procédé selon l'une des revendications précédentes, la régénération étant effectuée à une pression dans la plage de 0,5 à 200 bars.

9. Procédé selon l'une des revendications précédentes, la durée de la régénération étant d'au moins 24 heures.

10. Procédé selon l'une des revendications précédentes, le flux gazeux étant guidé, lors de la régénération, dans un sens d'écoulement identique ou inverse, par rapport au sens d'écoulement lors de l'hydrogénation, sur le catalyseur, de préférence dans le sens d'écoulement identique.
